# EUROPEAN PATENT APPLICATION

(11) **EP 2 856 962 A1**
(43) Date of publication of application: **08.04.2015**
(21) Application number: 13798226.0
(22) Date of filing: 31.05.2013
(51) Int. Cl.: A61B 18/04, A61B 18/12

(54) **ENERGY-USING TREATMENT TOOL**

(30) Priority: 01.06.2012 US 201261654431 P
(71) Applicant: OLYMPUS MEDICAL SYSTEMS CORP., Tokyo 151-0072 (JP)
(72) Inventor: SOBAJIMA, Hideo, Hachioji-shi Tokyo 192-8512 (JP); TAKASHINO, Tomoyuki, Hachioji-shi Tokyo 192-8512 (JP)
(74) Representative: Gunzelmann, Rainer
(86) International application number: PCT/JP2013/065262
(87) International publication number: WO 2013/180294

(57) **Abstract**

A treatment section of a surgical device, configured to apply energy to a biological tissue as a treatment target, and treat the biological tissue as the treatment target, includes: first and second jaws which are relatively openable/closable to each other to enable holding and releasing the biological tissue including the treatment target and its surrounding tissue; a pair of mutually opposed first holding sections which are provided respectively on inner sides of outer edges of the first and second jaws, and configured to hold the biological tissue as the treatment target; an energy discharge section which is provided on at least one of the pair of first holding sections, and configured to give energy for a treatment to the biological tissue as the treatment target; and a pair of second holding sections which are provided respectively between the first holding sections and the outer edges of the first and second jaws, and configured to hold the surrounding tissue of the biological tissue as the treatment target while bending the surrounding tissue with respect to the biological tissue as the treatment target.

## Description

### Technical field

The present invention relates to a surgical device configured to apply energy to a biological tissue as a treatment target, thereby treating the biological tissue.

### Background Art

For example, each of US 2006/0217709 A1 and US 6,500,176 B1 discloses a surgical device that can pinch a biological tissue to give a treatment. Of these examples, US 6,500,176 B1 discloses that a surface of an electrode and an outer surface of the same are formed into a continuous flat surface.

In general, when energy is applied to a biological tissue in a state that the biological tissue is held by a surgical device having an openable/closable treatment section, thereby treating the biological tissue, a fluid containing water vapor, a body fluid and the like is produced from the biological tissue. Such a fluid has a high temperature, and hence when it flows to the outside of the treatment section, the biological tissue is prone to thermal damage. Therefore, for example, in US 2008/0195091 A1, a groove is formed in a treatment section to surround an outer edge of an energy discharge section such as an electrode, whereby a fluid flows into a space formed by the groove.

When the outer edge portion has a wide lateral width, a region that pushes a biological tissue expands, and hence thermal damage is hardly brought about as compared with a case where the outer edge portion has a narrow lateral width. On the other hand, when the lateral width of the outer edge portion is increased, the treatment section can be enlarged.

### Summary of Invention

It is an object of the present invention to provide a surgical device that can suppress thermal damage of a surrounding tissue while preventing a lateral width of a treatment section from increasing.

One aspect of a surgical device including a treatment section according to the present invention, configured to apply energy to a biological tissue as a treatment target, and treat the biological tissue as the treatment target, wherein the treatment section includes: first and second jaws which are relatively openable/closable to each other to enable holding and releasing the biological tissue including the treatment target and its surrounding tissue; a pair of mutually opposed first holding sections which are provided respectively on inner sides of outer edges of the first and second jaws, and configured to hold the biological tissue as the treatment target; an energy discharge section which is provided on at least one of the pair of first holding sections, and configured to give energy for a treatment to the biological tissue as the treatment target; and a pair of second holding sections which are provided respectively between the first holding sections and the outer edges of the first and second jaws, and configured to hold the surrounding tissue of the biological tissue as the treatment target while bending the surrounding tissue with respect to the biological tissue as the treatment target.

### Brief Description of Drawings

FIG. 1 is a schematic view showing a treatment system using energy according to a first embodiment;
FIG. 2 is a schematic block diagram of the therapeutic treatment system according to the first embodiment;
FIG. 3 is a schematic view showing output states of energies from a high-frequency energy output circuit and a thermal energy output circuit as energy sources in the treatment system according to the first embodiment;
FIG. 4A is a schematic lateral cross-sectional view of a treatment section of a surgical device in the treatment system according to the first embodiment;
FIG. 4B is a schematic lateral cross-sectional view of the treatment section of the treatment device in the treatment system according to the first embodiment at a position indicated by reference numeral 4B in FIG. 4A;
FIG. 5A is a schematic lateral cross-sectional view showing a state that a biological tissue as a treatment target and its surrounding tissue are gripped by the treatment section of the surgical device in the treatment system according to the first embodiment;
FIG. 5B is a schematic lateral cross-sectional view showing a state that the biological tissue as the treatment target and its surrounding tissue are gripped by the treatment section of the surgical device in the treatment system according to the first embodiment at a position denoted by reference numeral 5B in FIG. 5A;
FIG. 6A is a schematic view showing that the surgical device according to the first embodiment is a bipolar type;
FIG. 6B is a schematic view showing that the surgical device according to the first embodiment is a monopolar type;
FIG. 7 is a schematic lateral cross-sectional view of a treatment section of a surgical device in a treatment system according to a first modification of the first embodiment;
FIG. 8 is a schematic lateral cross-sectional view of a treatment section of a surgical device in a treatment system according to a second modification of the first embodiment;
FIG. 9 is a schematic lateral cross-sectional view of a treatment section of a surgical device in a treatment system according to a third modification of the first embodiment;
FIG. 10 is a schematic lateral cross-sectional view of a treatment section of a surgical device in a treatment system according to a forth modification of the first embodiment;
FIG. 11 is a schematic lateral cross-sectional view of a treatment section of a surgical device in a treatment system according to a fifth modification of the first embodiment;
FIG. 12 is a schematic lateral cross-sectional view of a treatment section of a surgical device in a treatment system according to a sixth modification of the first embodiment;
FIG. 13 is a schematic view showing a treatment system using energy according to a second embodiment;
FIG. 14A is a schematic longitudinal cross-sectional view showing a state that a main body side treatment section and a separation side treatment section of a surgical device are engaged and the separation side treatment section is separated and opened with respect to the main body side treatment section in the treatment system according to the second embodiment;
FIG. 14B is a schematic longitudinal cross-sectional view showing a state that the main body side treatment section and the separation side treatment section of the surgical device are engaged and the separation side treatment section is moved closer and closed with respect to the main body side treatment section in the treatment system according to the second embodiment;
FIG. 14C is a schematic view showing a surface of the main body side treatment section of the surgical device in the treatment system according to the second embodiment;
FIG. 15A is a schematic longitudinal cross-sectional view showing a state that the separation side treatment section is moved closer and closed with respect to the main body side treatment section of the surgical device in the treatment system according to the second embodiment at a position denoted by reference numeral 15A in FIG. 14B;
FIG. 15B is a schematic longitudinal cross-sectional view showing a state that a separation side treatment section is moved closer and closed with respect to a main body side treatment section of a surgical device in a treatment system according to a first modification of the second embodiment at the position denoted by reference numeral 15A in FIG. 14B;
FIG. 15C is a schematic longitudinal cross-sectional view showing a state that a separation side treatment section is moved closer and closed with respect to a main body side treatment section of a surgical device in a treatment system according to a second modification of the second embodiment at the position denoted by reference numeral 15A in FIG. 14B;
FIG. 15D is a schematic longitudinal cross-sectional view showing a state that a separation side treatment section is moved closer and closed with respect to a main body side treatment section of a surgical device in a treatment system according to a third modification of the second embodiment at the position denoted by reference numeral 15A in FIG. 14B; and
FIG. 15E is a schematic longitudinal cross-sectional view showing a state that a separation side treatment section is moved closer and closed with respect to a main body side treatment section of a surgical device in a treatment system according to a fourth modification of the second embodiment at the position denoted by reference numeral 15A in FIG. 14B.

### Description of Embodiments

Modes for carrying out the present invention will now be described hereinafter in detail with reference to the drawings.

### [First Embodiment]

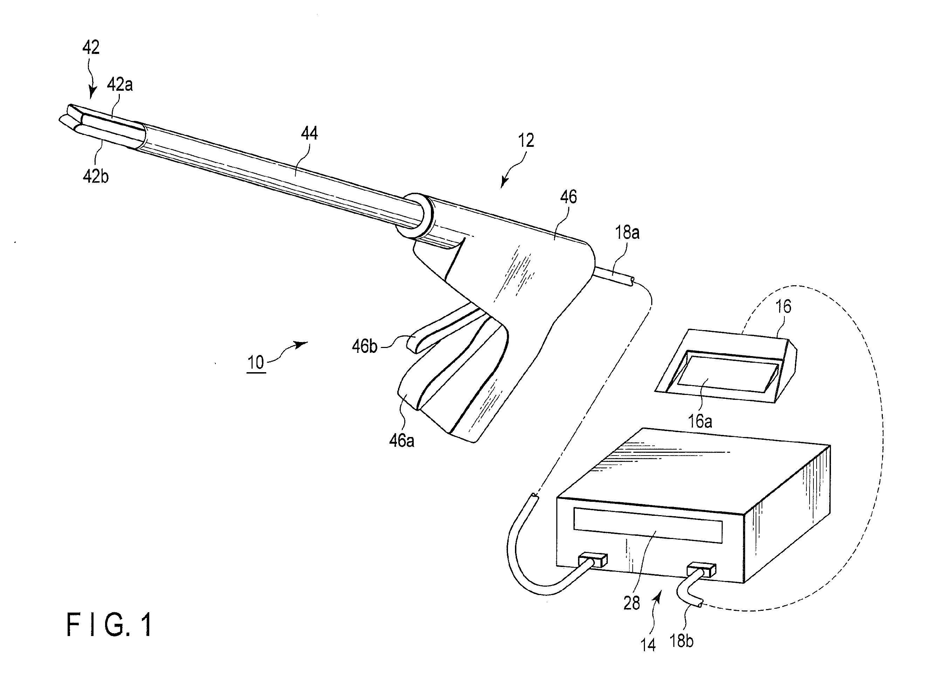

A first embodiment will now be described with reference to FIG. 1 to FIG. 6B.

As shown in FIG. 1, a treatment system 10 using energy according to this embodiment includes a surgical device (an energy treatment device) 12 and an energy source 14 that gives energy to the surgical device 12. A foot switch 16 having a pedal 16a that switches ON/OFF of the energy given to the surgical device 12 is connected to the energy source 14. The surgical device 12 is electrically connected to the energy source 14 through a first cable 18a formed by bundling lead wires or signal wires, and the energy source 14 is electrically connected to the foot switch 16 through a second cable 18b formed by bundling lead wires or signal wires. The foot switch 16 can input a signal to the energy source 14 by, e.g., an operation of the pedal 16a, and the energy source 14 can control the energy given to the treatment device 12 based on, e.g., an operation of the pedal 16a of the foot switch 16.

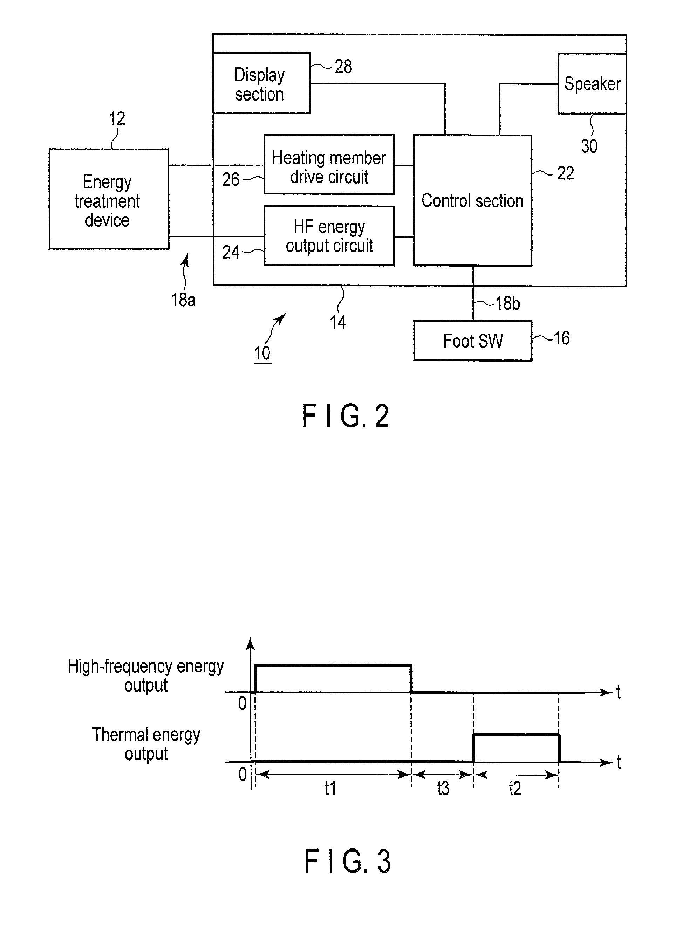

As shown in FIG. 2, the energy source 14 has a control section 22, a high-frequency energy output circuit 24, a heating member drive circuit 26, a display section 28, and a speaker 30.

Here, the high-frequency energy output circuit 24 in the energy source 14 is controlled by the control section 22 so that it gives high-frequency energy to later-described electrodes 72 and 74 of the treatment device 12 to generate heat from a biological tissue L held between the electrodes 72 and 74, and the biological tissue L is denatured by this thermal energy. The heating member drive circuit 26 in the energy source 14 is controlled by the control section 22 so that it supplies energy to heating members (resistance heating heaters) 82 and 84 to generate heat therefrom, transfers this heat (thermal energy) to the electrodes 72 and 74, and also transfers the heat (the thermal energy) to the biological tissue L to dehydrate the biological tissue L. That is, the surgical device 12 according to this embodiment enables the thermal energy to act on the biological tissue L and gives a treatment to the biological tissue L.

As the display section 28, it is preferable to use, e.g., a touch panel so that it can be used for displaying a state of the energy source 14 or configuring various kinds of settings. Further, the speaker 30 is controlled so that it can inform of ON/OFF of an output from the high-frequency energy output circuit 24 or the heating member drive circuit 26 with the use of sound.

The control section 22 of the energy source 14 can control a supply time and so on in the case of supplying high-frequency energy (thermal energy) using the later-described electrodes 72 and 74 of the treatment device 12 and the thermal energy using the later-described heating members 82 and 84 to the biological tissue L. The control section 22 controls the high-frequency energy output circuit 24 to output the appropriate high-frequency energy for a time t1 by pressing down the pedal 16a of the foot switch 16 and to stop the output as shown in FIG. 3, and then controls the speaker 30 to generate the sound so that an operator can be informed of end of a treatment using the later-described electrodes 72 and 74. Furthermore, the control section 22 gives a treatment using the high-frequency energy, then controls the heating member drive circuit 26 to output the appropriate thermal energy for a time t2 and to stop the output, and thereafter controls the speaker 30 to generate the sound so that the operator can be informed of the end of the treatment using the later-described heating members 82 and 84. It is to be noted that a time t3 during which the treatment using the high-frequency energy is switched to the treatment using the thermal energy may be 0, or an appropriate time, e.g., several seconds may be taken.

The control section 22 may switch the setting for outputting the appropriate high-frequency energy for the time t1 with the use of the high-frequency energy output circuit 24 to a setting for outputting the high-frequency energy using a change in biological information (e.g., impedance or a phase difference) of the measurable biological tissue L by the electrodes 72 and 74 based on the setting in the display section 28, and the output of the high-frequency energy may be stopped when one of both the members (the time and the biological information) is reached ahead of the other.

As shown in FIG. 1, the surgical device 12 includes a treatment section 42 that gives a treatment to the biological tissue L, an inserting section 44, and an operating section 46.

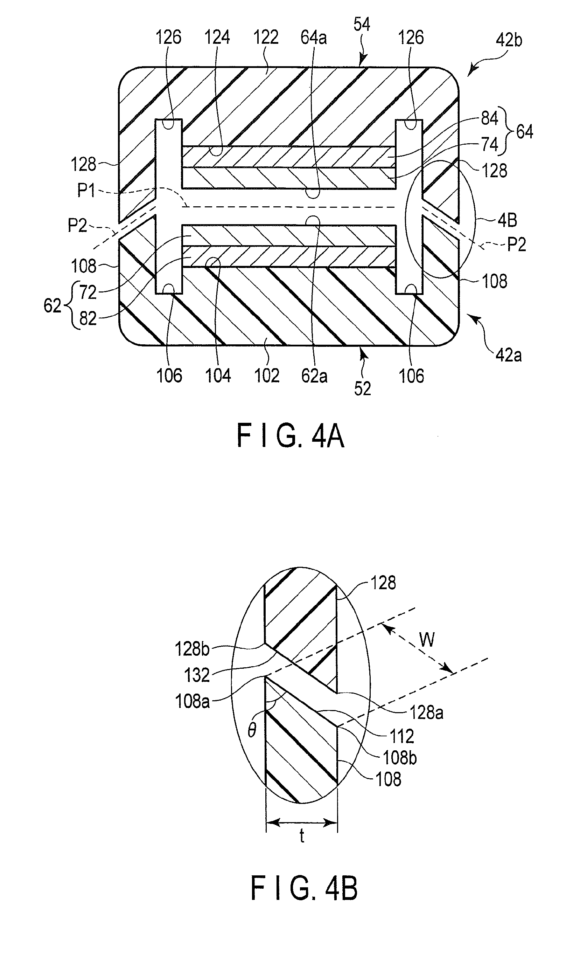

As shown in FIG. 4A, the treatment section 42 includes a pair of openable/closable jaws (first and second jaws) 52 and 54 as a holding section for a biological tissue, and energy discharge sections 62 and 64 arranged on the jaws 52 and 54. In this embodiment, the energy discharge sections 62 and 64 are depicted to have a flat plate shape in FIG. 4A, but various shapes are acceptable. It is to be noted that, for example, ceramics, a resin having heat-resisting properties and insulation properties, an insulated metal material, or the like is appropriately used for the first and second jaws 52 and 54.

Opening/closing of the first and second jaws 52 and 54 shown in FIG. 1, i.e., opening/closing of first and second treatment sections 42a and 42b is operated by an opening/closing lever 46a in the operating section 46. When the opening/closing lever 46a is operated, the first and second jaws 52 and 54 are opened/closed by well-known means such as a wire or a rod arranged in the inserting section 44. It is to be noted that one of the first and second jaws 52 and 54 alone may be configured to move, or both of them may be configured to move. That is, the first and second jaws 52 and 54 can be relatively opened/closed. In this embodiment, a description will be given as to an example in which, with respect to one (the first jaw 52) of the first and second jaws 52 and 54, the other (the second jaw 54) is configured to move.

As shown in FIG. 4A, the energy discharge sections 62 and 64 have the high-frequency electrodes 72 and 74 and the heating members 82 and 84 that are arranged on the high-frequency electrodes 72 and 74, respectively. Of these members, the first jaw 52, the high-frequency electrode 72, and the heating member 82 (the first energy discharge section 62) form the first treatment section 42a, and the second jaw 54, the high-frequency electrode 74, and the heating member 84 (the second energy discharge section 64) form the second treatment section 42b.

Heating elements may be used for each of the heating members 82 and 84, and a plate-like heater may be used for the same. It is preferable to arrange or bury the heating members 82 and 84 on or in back surfaces of the electrodes 72 and 74 if each of the heating members 82 and 84 is formed of the heating elements and preferable to arrange the heating members 82 and 84 on the back surfaces of the electrodes 72 and 74 if each of the heating members 82 and 84 is the plate-like heater. It is also preferable for each of the heating members 82 and 84 to have a bar shape that is long in a longitudinal direction of the electrodes 72 and 74 or a direction orthogonal to the longitudinal direction.

The high-frequency electrodes 72 and 74 face each other and are used as holding surfaces 62a and 64a for the biological tissue L as the treatment target. That is, the holding surfaces (a first holding section) 62a and 64a are formed as a holding section (the first holding section) of the biological tissue L as the treatment target. Therefore, when the high-frequency energy is given to the electrodes 72 and 74 in a state that the biological tissue L is held between the holding surfaces 62a and 64a of the electrodes 72 and 74, the biological tissue L can be denatured by the thermal energy that has heated the biological tissue L. Further, the electrodes 72 and 74 are made of a material having excellent thermal conductivity. Therefore, when heat is generated from the heating members 82 and 84, the heat (thermal energy) is transferred to the electrodes 72 and 74, and the heat (the thermal energy) can be further transferred to the biological tissue L held between the holding surfaces 62a and 64a of the electrodes 72 and 74. Therefore, the holding surfaces 62a and 64a also function as treatment surfaces for the biological tissue L.

The first jaw (the lower jaw) 52 of the treatment section 42 in this embodiment is, e.g., a fixed type, and the second jaw (the upper jaw) 54 is a movable type that can be opened/closed with respect to the first jaw 52.

Each of the first and second jaws 52 and 54 is formed into a substantially tabular shape that is long in a direction (a longitudinal direction) parallel to the longitudinal direction of the inserting section 44 and has a width direction orthogonal to the longitudinal direction formed to be smaller than the longitudinal direction.

The first jaw 52 has a main body 102, a concave portion 104 which is formed on the inner side of an outer edge of a main body 102 (the center in the longitudinal direction and the width direction is preferable) and in which the energy discharge section 62 (i.e., the electrode 72 and the heating member 82) is arranged, a groove 106 which is formed in the main body 102 and arranged to surround an outer side of the concave portion 104, and an outer edge portion (a barrier portion) 108 that is formed in the main body 102 and arranged to surround an outer side of the groove 106. The concave portion 104, the groove portion 106, and the outer edge portion 108 are formed at positions where they face the second jaw 54. It is to be noted that the outer edge portion 108 may be integrally formed with the main body 102 of the first jaw 52 or may be formed as a separate body. The outer edge portion 108 forms a holding section (a second holding section) that faces a later-described outer edge portion 128 and holds a surrounding tissue S of the biological tissue L as the treatment target.

In this embodiment, although the surface 62a of the electrode 72 is formed as a flat surface, it may have irregularities.

As shown in FIG. 4B, the outer edge portion 108 itself has an end portion (the uppermost end) 108a on its inner side (a side close to the energy discharge section 62) placed above an edge portion 108b on its outer side (a side apart from the energy discharge section 62), and the end portion 108a on the inner side and the end portion 108b on the other side are smoothly continuous with each other. Therefore, a surface, which faces the later-described outer edge portion 128 of the second jaw 54, of the outer edge portion 108 of the first jaw 52 is formed as an inclined surface (a second holding surface) 112.

The groove 106 is used for receiving, in cooperation with the outer edge portion 108, a later-described liquid produced in the biological tissue L as the treatment target when energy is applied to the biological tissue L as the treatment target. It is to be noted that the groove 106 is closed or communicates on a distal end side of the first jaw 52, and it is opened on a proximal end side of the first jaw 52. That is, the groove 106 can allow the later-described fluid to flow to the proximal end side (the inserting section 44 side) of the treatment section 42.

As shown in FIG. 4A, the second jaw 54 includes a main body 122, a concave portion 124 which is formed on the inner side of an outer edge of the main body 122 (the center in the longitudinal direction and the width direction is preferable) and in which the energy discharge section 62 (i.e., the electrode 74 and the heating member 84) is arranged, a groove 126 which is formed in the main body 122 and arranged on the outer side of the concave portion 124, and an outer edge portion (a barrier portion) 128 that is formed in the main body 122 and arranged on the outer side of the groove 126. The concave portion 124, the groove portion 126, and the outer edge portion 128 are formed at positions where they face the first jaw 52. It is to be noted that the outer edge portion 128 may be integrally formed with the main body 122 of the second jaw 54 or may be formed as a separate body.

Further, the energy discharge sections 62 and 64, the groove portions 106 and 126, and the outer edge portions 108 and 128 of the first and second jaws 52 and 54 face each other, and they move closer to or away from each other by moving the first and second jaws 52 and 54 closer or away from each other. It is to be noted that the outer edge portions 108 and 128 facing each other may abut on each other or may have a gap therebetween and not abut on each other when the first and second jaws 52 and 54 are closed each other in a state that a biological tissue is not held. The energy discharge sections 62 and 64 and the gap portions 106 and 126 that face each other are apart from each other when the first and second jaws 52 and 54 are closed in a state that a biological tissue is not held. That is, the first and second jaws 52 and 54 are formed in such a manner that the surfaces 62a and 64a of the electrodes 72 and 74 do not come into contact with each other when the first and second jaws 52 and 54 are closed.

In this embodiment, the surface 64a of the electrode 74 is formed as a flat surface, but it may have irregularities.

As shown in FIG. 4B, the outer edge portion 128 itself includes an end portion 128b on its inner side (a side close to the energy discharge section 64) placed above an end portion (the lowermost end) 128a on its outer side (a side apart from the energy discharge section 64), and the end portion 128b on the inner side and the end portion 128a on the other side are smoothly continuous with each other. Therefore, a surface, which faces the outer edge portion 108 of the first jaw 52, of the outer edge portion 128 of the second jaw 54 is formed as an inclined surface (a second holding surface) 132. In addition, it is preferable for the inclined surface 112 of the outer edge portion 108 in the first jaw 52 to be parallel to or substantially parallel to the inclined surface 132 of the outer edge portion 128 in the second jaw 54.

The groove 126 is used for receiving, in cooperation with the outer edge portion 128, the later-described liquid produced when energy is applied to the biological tissue L as the treatment target. It is to be noted that the groove 126 is closed or communicates on a distal end side of the second jaw 54, and it is opened on a proximal end side of the second jaw 54. That is, the groove 126 can allow the later-described fluid to flow to the proximal end side (the inserting section 44 side) of the treatment section 42.

Here, as shown in FIG. 4B, assuming that a thickness of the outer edge portion 108 of the first jaw 52 is t and an angle of the inclined surface 112 relative to an inner surface of the outer edge portion 108 is θ (0 degrees<θ<90 degrees), a width W of the inclined surface 112 of the outer edge portion 108 can be expressed as W=t/sinθ. That is, when the angle θ of the inclined surface 112 is 90 degrees, the width W of the inclined surface 112 of the outer edge portion 108 coincides with the thickness t of the outer edge portion 108. Therefore, the width W of the inclined surface 112 of the outer edge portion 108 according to this embodiment is t/sinθ larger in accordance with the angle θ of the inclined surface 112 than that in a case where the angle θ is 90 degrees. Therefore, when the inclined surfaces 112 and 132 are formed, a contact area of the biological tissue L as the treatment target with respect to the surrounding tissue S can be increased as compared with a case where the angle θ is 90 degrees.

It is to be noted that the outer edge portion 128 of the second jaw 54 is configured to face the outer edge portion 108 of the first jaw 52. Therefore, a detailed description of the outer edge portion 128 of the second jaw 54 will be omitted.

A function of the treatment system 10 according to this embodiment will now be described.

For example, the treatment section 42 is placed to face the biological tissue L as the treatment target. In this state, the opening/closing lever 46a of the operating section 46 is operated to hold the biological tissue L between the holding surfaces 62a and 64a of the energy discharge sections 62 and 64.

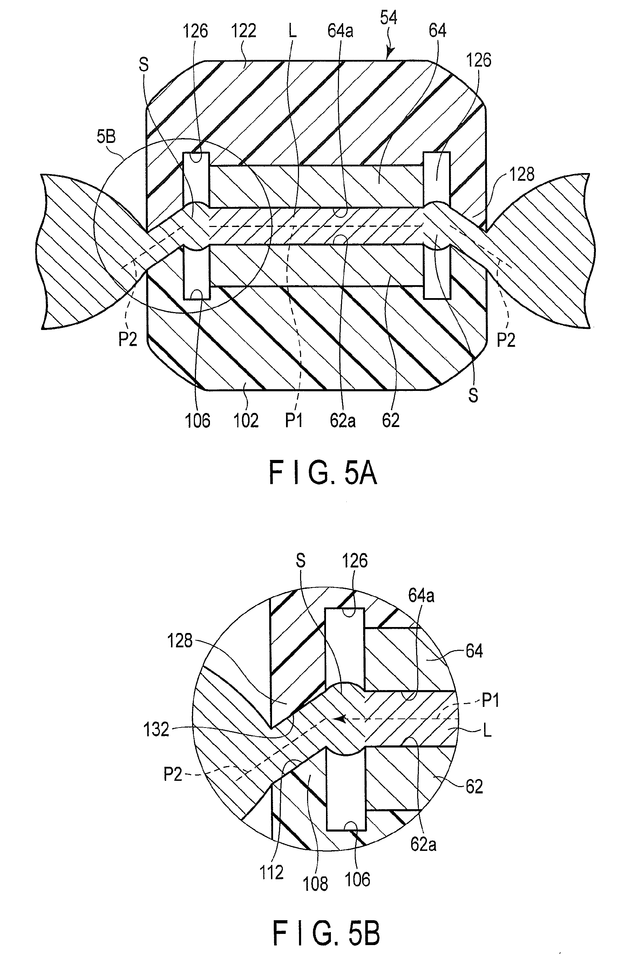

As shown in FIG. 5A, when the biological tissue L as the treatment target and its surrounding tissue S are held by the first and second treatment sections 42a and 42b, the biological tissue L as the treatment target is arranged to be appressed against the surfaces 62a and 64a of the energy discharge sections 62 and 64 and also arranged between the grooves 106 and 126. At this time, when midpoints between the surfaces 62a and 64a of the energy discharge sections 62 and 64 are collected, a first virtual plane P1 can be defined. That is, the first virtual plane P1 is defined midway between the surfaces 62a and 64a of the energy discharge sections 62 and 64. This virtual plane P1 is a substantially flat plane (including a flat plane) that is substantially parallel (being parallel is included) to the surfaces 62a and 64a of the energy discharge sections 62 and 64.

When the biological tissue is held by the first and second treatment sections 42a and 42b, the surrounding tissue S of the biological tissue L as the treatment target is arranged between the grooves 106 and 126 and the outer edge portions 108 and 128. At this time, when midpoints between the inclined surfaces 112 and 132 of the outer edge portions 108 and 128 are collected, a second virtual plane P2 can be defined as shown in FIG. 5B. This surface (the second virtual plane defined midway between the inclined surfaces 112 and 132 of the outer edge portions 108 and 128) forms an inclined surface that is inclined with respect to the first virtual plane P1 between the surfaces 62a and 64a of the energy discharge sections 62 and 64. It is to be noted that, in this embodiment, this virtual plane P2 is a substantially flat surface (including a flat surface), and the virtual plane P2 becomes a curved surface if the inclined surfaces 112 and 132 are formed as curved surfaces that are substantially parallel (being parallel is included) to each other.

As described above, when the biological tissue is held by the first and second treatment sections 42a and 42b, the biological tissue L as the treatment target is arranged along the virtual plane P1 between the surfaces 62a and 64a of the energy discharge sections 62 and 64. Therefore, when the biological tissue L is held by the first and second treatment sections 42a and 42b, the surrounding tissue S of the biological tissue L as the treatment target is bent at a boundary between virtual plane P1 between the surfaces 62a and 64a of the energy discharge sections 62 and 64 and the virtual plane P2 between the grooves 106 and 126. In other words, a shear force that does not lead to cutting is applied to the surrounding tissue S of the biological tissue L as the treatment target, and the surrounding tissue S is bent.

That is, the surrounding tissue S of the biological tissue L as the treatment target held between the outer edge portions (the second holding section) 108 and 128 arranged outside the holding surfaces (the first holding section) 62a and 64a can be bent with respect to the biological tissue L as the treatment target held between the holding surfaces (the first holding section) 62a and 64a provided on the central side (the inner side) relative to each outer edge of the first and second jaws 52 and 54.

As described above, when the surrounding tissue S is held between the inclined surfaces 112 and 132 of the outer edge portions 108 and 128 while being bent, the contact area relative to the surrounding tissue S can be increased, the surrounding tissue S is held in a state that it is pressed by the inclined surfaces 112 and 132 while applying the shear force that does not result in cutting, and hence the biological tissue hardly slips on the inclined surfaces 112 and 132 even though the biological tissue is pulled, thereby stably holing the surrounding tissue S.

It is to be noted that, when the biological tissue is held by the first and second treatment sections 42a and 42b, part of the surrounding tissue S of the biological tissue L as the treatment target enters the grooves 106 and 126.

Therefore, when the biological tissue is held by the first and second treatment sections 42a and 42b, the surrounding tissue S of the biological tissue L as the treatment target is held between the inclined surfaces 112 and 132 of the first and second outer edge portions 108 and 128 in a pressed state. Therefore, a region surrounded by the biological tissue L as the treatment target, the surrounding tissue S, the outer edge portion 108 provided to the first jaw 52, the groove portion 106, and the holding surface 62a of the energy discharge section 62 is closed. Likewise, a region surrounded by the biological tissue L as the treatment target, the surrounding tissue S, the outer edge portion 128 provided to the second jaw 54, the groove portion 126, and the holding surface 64a of the energy discharge section 64 is closed.

When a state that the pedal 16a of the foot switch 16 is pressed down with a foot is maintained in this situation, the control section 22 of the energy source 14 gives energy to the high-frequency electrodes 72 and 74 from the high-frequency energy output circuit 24. Therefore, the biological tissue L between the surfaces 62a and 64a of the electrodes 72 and 74 is heated by thermal energy (Joule heat) produced from the high-frequency energy. Further, the biological tissue L is denatured by the thermal energy, and then supply of the energy to the high-frequency electrodes 72 and 74 is stopped. It is to be noted that the control section 22 of the energy source 14 gives the energy to the biological tissue L between the high-frequency electrodes 72 and 74 for the predetermined time t1 and then stops output of the energy from the high-frequency energy output circuit 24.

Here, when the predetermined time t1 has passed from the start of outputting the energy, the energy source 14 stops the supply of the energy to the high-frequency electrodes 72 and 74 even though the pedal 16a of the foot switch 16 is being depressed. On the other hand, when a foot is released from the pedal 16a before the predetermined time t1 passes, the energy source 14 stops the supply of the energy to the high-frequency electrodes 72 and 74 from the moment of release.

As described above, when the biological tissue L as the treatment target between the surfaces 62a and 64a of the energy discharge sections 62 and 64 is heated, a fluid such as water vapor (a gas) or a biological fluid (a liquid) is produced from the biological tissue L that is in contact with or appressed against the surfaces 62a and 64a. At this time, since the region surrounded by the biological tissue L, the outer edge portion 108, the groove 106, and the surface 62a of the energy discharge section 62 is formed as a closed space, its inner pressure is raised. Therefore, the fluid flows toward the groove 106 along the surface 62a of the energy discharge section 62, i.e., the surface of the biological tissue L and flows into the groove 106. Likewise, since the region surrounded by the biological tissue L, the outer edge portion 128, the groove 126, and the surface 64a of the energy discharge section 64 is formed as a closed space, its inner pressure is raised. Therefore, the fluid flows toward the groove 126 along the surface 64a of the energy discharge section 64, i.e., the surface of the biological tissue L and flows into the groove 126.

As described above, the inner pressure of the region surrounded by the biological tissue L, the outer edge portion 108, the groove 106, and the surface 62a of the energy discharge section 62 and the inner pressure of the region surrounded by the biological tissue L, the outer edge portion 128, the groove 126, and the surface 64a of the energy discharge section 64 are raised. Therefore, part of the fluid is to flow from the inner side toward the outer side of the inclined surface 112 and 132 through the inclined surfaces 112 and the 132 of the outer edge portions 108 and 128.

Here, the virtual plane P2 between the inclined surfaces 112 and 132 of the outer edge portions 108 and 128 is not formed as a plane (the same plane) continuous with the virtual plane P1 between the surfaces 62a and 64a of the energy discharge sections 62 and 64 but as a slant plane. Therefore, as compared with a case where the virtual plane (the slant plane) P2 is flush (the same plane) with the virtual plane (the flat plane) P1, a length (a width W of the slant plane) from the inner side to the outer side of each of the outer edge portions 108 and 128 can be increased. As described, since the length (the width W of the slant plane), i.e., a path from the inner side to the outer side of each of the outer edge portions 108 and 128 can be increased, discharge of the heat from the treatment section 42 to the outside can be more effectively prevented, and spreading of heat from the biological tissue L as the treatment target to its surrounding tissue S can be suppressed, i.e., thermal damage to the surrounding tissue S can be suppressed. Therefore, the fluid can be more effectively prevented from being discharged to the outside of the first and second jaws 52 and 54, and the fluid can be effectively collected in the grooves 106 and 126.

Furthermore, when the virtual plane P2 is formed as the slant plane as described above, the thickness t of each of the outer edge portions 108 and 128 does not have to be changed. Therefore, a lateral width of each of the jaws 52 and 54 does not have to be increased, and insertability can be excellently maintained at the time of inserting the treatment section 42 to the biological tissue as the treatment target.

Moreover, the surrounding tissue S of the biological tissue L as the treatment target that is present on the virtual plane P2 between the inclined surfaces 112 and 132 of the outer edge portions 108 and 128 is bent near a position between the grooves 106 and 126 with respect to the biological tissue L as the treatment target that is present on the virtual plane P1. That is, the surrounding tissue S can be bent near the boundary between the virtual planes P1 and P2 with respect to the biological tissue L as the treatment target. Therefore, the energy is applied to the biological tissue L as the treatment target, the fluid is produced from the biological tissue S as the treatment target, and a flowing direction of the fluid can be changed at a position where the surrounding tissue S is bent with respect to the biological tissue L as the treatment target when the fluid flows along the surfaces 62a and 64a of the energy discharge sections 62 and 64. As described above, since the surrounding tissue S is bent with respect to the biological tissue L as the treatment target, an intense flow path resistance can be applied to the fluid as compared with a case where the biological tissue L as the treatment target and its surrounding tissue S have flat surfaces that are flush with each other. That is, since the surface along which the fluid flows is bent, the force of flow of the fluid can be weakened as compared with a case where a surface along which the fluid flows is a flat surface.

Moreover, although the inner pressure of each of the regions surrounded by the biological tissue, the outer edge portions 108 and 128, the grooves 106 and 126, and the surfaces 62a and 64a of the energy discharge sections 62 and 64 is increased, the outer edge portions 108 and 128 facing each other stably exercise a holding force (pressing force) for holding the surrounding tissue S. Therefore, heat generated when the biological tissue L as the treatment target is subjected to a treatment can be prevented from being discharged to the outside of the treatment section 42.

It is to be noted that the surrounding tissue S of the biological tissue L as the treatment target which has entered the grooves 106 and 126 is affected by the high-frequency energy at a region closer to the high-frequency electrodes 72 and 74. Therefore, of the surrounding tissue S that has entered the grooves 106 and 126, a part that has come into contact with the high-frequency electrodes 72 and 74 is subjected to a treatment together with the biological tissue L as the treatment target. Additionally, the fluid produced from the biological tissue L between the surfaces 62a and 64a of the electrodes 72 and 74 has a higher temperature than the surrounding tissue S and the fluid moves along the surfaces of the biological tissue L and its surrounding tissue S, the surrounding tissue S that has entered the grooves 106 and 126 is apt to be influenced by the spreading of heat. However, as the surrounding tissue S is firmly held between the end portion 108a of the outer edge portion 108 and the end portion 128b of the outer edge portion 128, the inner edge portions 108a and 128b of the outer edge portions 108 and 128 exercise a function of a barrier section that prevents the fluid from moving to the outside.

Further, when the state that the pedal 16a of the foot switch 16 is being depressed with a foot is maintained, the output of the energy from the high-frequency energy output circuit 24 is stopped, an appropriate time (a time t3 in FIG. 3) passes (it may be 0 second), and then the energy is output from the heating member drive circuit 26 to heat the heating members 82 and 84 for a time t2. Therefore, heat (thermal energy) of the heating members 82 and 84 is transferred to the electrodes 72 and 74, and the biological tissue L can be dehydrated. At this time, a fluid is generated from the biological tissue L as the treatment target, and would normally flow toward the outside of the treatment section 42; but the fluid flows into the grooves 106 and 126, and the surrounding tissue S is pressed by the inclined surfaces 112 and 132 of the outer edge portions 108 and 128, thereby suppressing the thermal spread.

As described above, the following can be said.

When the biological tissue L as the treatment target and its surrounding tissue S are held by the first and second treatment sections 42a and 42b, the surrounding tissue S between the outer edge portions 108 and 128 can be bent with respect to the biological tissue L between the surfaces 62a and 64a of the energy discharge sections 62 and 64 and the grooves 106 and 126. As described above, adopting the configuration in which the surrounding tissue S can be bent with respect to the biological tissue L as the treatment target enables changing a direction along which the fluid discharged from the biological tissue L flows under the influence of the energy discharge sections 62 and 64 from a direction parallel to the virtual plane P1 between the surfaces 62a and 64a of the energy discharge sections 62 and 64 to a direction parallel to the virtual plane P2 between the inclined surfaces 112 and 132 of the outer edge portions 108 and 128. Therefore, in the vicinity of the boundary between the plane P1 and the plane P2 in particular, a stronger flow path resistance can be exercised and the force of the fluid discharged from the biological tissue L can be weakened as compared with the case where the plane P1 and the plane P2 are the same flat plane.

Further, since the outer edge portions 108 and 128 are formed as the inclined surfaces 112 and 132 that are inclined with respect to the parallel-plane type surfaces (the holding surfaces) 62a and 64a of the electrodes 72 and 74, areas of the outer edge portions 108 and 128 can be increased as compared with a case where the outer edge portions 108 and 128 are parallel to the surfaces 62a and 64a of the electrodes 72 and 74. Therefore, the length (the width W of each of the inclined surfaces 112 and 132) from the inner side to the outer side of each of the outer edge portions 108 and 128 can be increased without changing the width (the thickness t) of each of the outer edge portions 108 and 128. Therefore, the path from the outer edge of each of the energy discharge sections 62 and 64 to the outer edge (an outer periphery of the treatment section 42) of each of the outer edge portions 108 and 128 can be increased without changing the lateral width of each of the first and second jaws 52 and 54. Therefore, the spreading of heat can be efficiently suppressed without changing the thickness of each of the outer edge portions 108 and 128.

It is preferable for the thicknesses t of the outer edge portions 108 and 128 of the first and second jaws 52 and 54 to be substantially equal to each other, but it is good enough to form the inclined surfaces 112 and 132 of the outer edge portions 108 and 128 of the first and second jaws 52 and 54 to face each other.

In this embodiment, although the description has been given as to the first jaw 52 that is the fixed jaw and the second jaw 54 that is the movable jaw, adopting the same configuration for the treatment sections that are movable jaws in the treatment device is preferable.

In this embodiment, although the description has been given as to the example where the heating members 82 and 84 are arranged on the back surfaces of the high-frequency electrodes 72 and 74, the heating members 82 and 84 may not be provided. That is, it is also preferable to perform a series of treatments for the biological tissue by using the high-frequency energy alone. In this case, removing the heating member drive circuit 26 from the energy source 14 shown in FIG. 2 is also preferable.

Furthermore, although the description has been given as to the example using the high-frequency electrodes 72 and 74, it is also preferable to use the high-frequency electrodes 72 and 74 as heat transfer members that transfer heat generated at the time of heating the heating members 82 and 84 to the biological tissue L in place of using them as members that produce the high-frequency energy. That is, the control section 22 of the energy source 14 shown in FIG. 2 may drive the heating member drive circuit 26 alone in regard to the series of treatments for the biological tissue L as the treatment target without using the high-frequency energy output circuit 24. In this case, it is also preferable to remove the high-frequency energy output circuit 24 from the energy source 14 shown in FIG. 2.

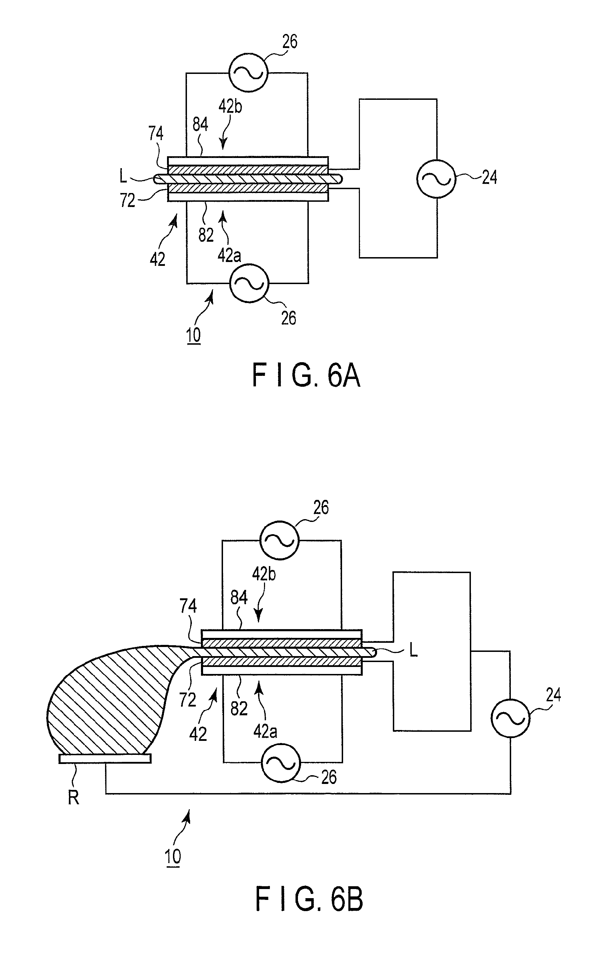

Moreover, in this embodiment, although the description has been given as to a case where the treatment device 12 is a bipolar type treatment device shown in FIG. 6A, the treatment device 12 may be used as a monopolar type treatment device shown in FIG. 6B. In the case of FIG. 6B, a treatment is given in a state that a counter electrode plate R is disposed to a patient P. That is, both the monopolar type and the bipolar type can be adopted for the treatment of the biological tissue L using the high-frequency electrodes 72 and 74. Additionally, in the case of using the treatment device 12 according to this embodiment as the monopolar type, the high-frequency energy may be given to one of the high-frequency electrodes 72 and 74 alone arranged in the pair of jaws 52 and 54. It is to be noted that, like the states shown in FIG. 6A and FIG. 6B, using tabular heaters as the heating members 82 and 84 is preferable. That is, arranging the heating members 82 and 84 on the back surfaces of the electrodes 72 and 74 is also preferable.

### [First Modification of First Embodiment]

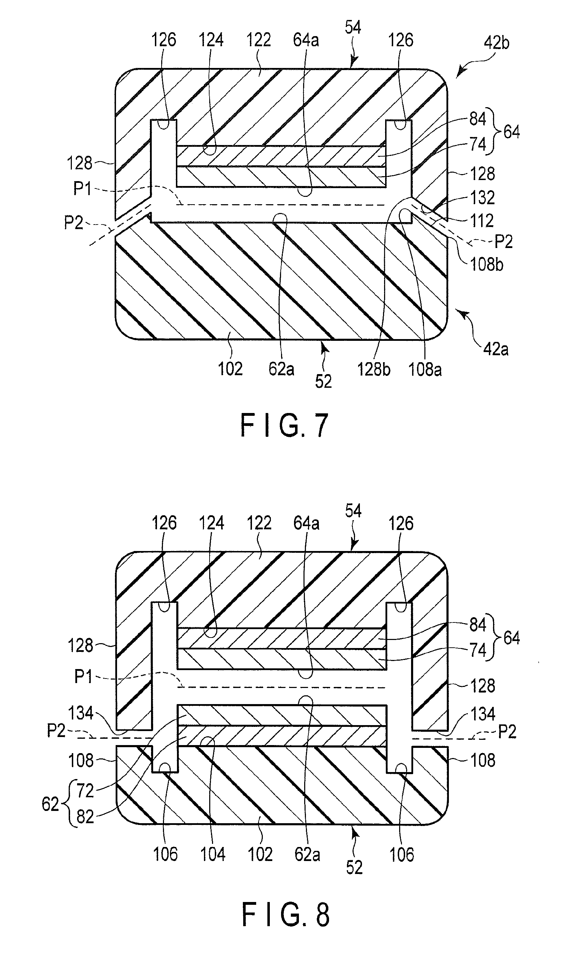

A first modification of the first embodiment will now be described with reference to FIG. 7. It is to be noted that like reference numerals denote the same members or members having the same functions as the members described in the first embodiment as much as possible, and a detailed description thereof will be omitted.

This modification is an example in which the energy discharge section 62 (the high-frequency electrode 72 and the heating member 82) and the groove 106 are omitted from the first jaw 52. Therefore, the concave portion 104 is omitted from the first jaw 52, and the holding surface 62a which holds the biological tissue L as the treatment target in cooperation with the holding surface 64a of the energy discharge section 64 of the second jaw 54 is formed on the first jaw 52 itself.

It is to be noted that, in this modification, the inner end portion 108a of the outer edge portion 108 of the first jaw 52 is preferably placed above the holding surface 62a. Therefore, the inner surface (the end portion 108a) of the outer edge portion 108 of the first jaw 52 functions as a barrier section that prevents the fluid from flowing to the outside of the treatment section 42 when the energy is applied to the biological tissue L as the treatment target from the energy discharge section 64.

It is to be noted that, in this embodiment, the high-frequency electrode 74 arranged in the concave portion 124 of the second jaw 54 is of the monopolar type. Therefore, the counter electrode plate R shown in FIG. 6B is disposed to a patient and then a treatment is given.

A function of the treatment system 10 using the energy according to this embodiment will now be briefly explained.

The biological tissue L as the treatment target is arranged between the holding surfaces 62a and 64a shown in FIG. 7, and the surrounding tissue S is arranged between the inclined surfaces 112 and 132 of the outer edge portions 108 and 128. Therefore, the surrounding tissue S is held in a bent state by the treatment section 42 with respect to the biological tissue L as the treatment target. When the energy is applied to the electrode 74 from the high-frequency energy output circuit 24 in this state, the biological tissue L as the treatment target is denatured, and the fluid is discharged from the biological tissue L. This fluid moves toward the outer edge portions 108 and 128 along the holding surfaces 62a and 64a. At this time, the inner sides of the outer edge portions 108 and 128 function as the barrier sections. Therefore, the fluid flows into the groove 126. Furthermore, since the surrounding tissue S is held on the inclined surfaces 112 and 132 each having a width larger than the thickness t of each of the outer edge portions 108 and 128, the spreading of heat can be effectively suppressed.

Even when the energy is output from the heating member drive circuit 26 to the heating member 84 after stopping the output to the high-frequency electrode 74 from the high-frequency energy output circuit 24, the spreading of heat can be likewise effectively suppressed.

### [Second Modification of First Embodiment]

A second modification of the first embodiment will now be described with reference to FIG. 8.

As shown in FIG. 8, this modification is an example in which the first virtual plane P1 between the surfaces 62a and 64a of the energy discharge sections 62 and 64 is set to be parallel or substantially parallel to the second virtual surface P2 between the outer edge portions 108 and 128 and these planes are shifted in a vertical direction (the opening/closing direction of the jaws 52 and 54). In this modification, the outer edge portions 108 and 128 are not formed as the inclined surfaces 112 and 132 shown in FIG. 4B but they are formed as surfaces 114 and 134 parallel to the first virtual plane P1. When the first and second virtual planes P1 and P2 are shifted in the vertical direction (the opening/closing direction of the jaws 52 and 54) as described above, the surrounding tissue S can be bent with respect to the biological tissue L as the treatment target.

As described above, when the configuration that the surrounding tissue S can be bent with respect to the biological tissue L as the treatment target is adopted, a flowing direction of the fluid discharged from the biological tissue L under the influence of the energy discharge sections 62 and 64 can be changed from a direction parallel to the virtual plane P1 between the surfaces 62a and 64a of the energy discharge sections 62 and 64 to a direction parallel to the virtual plane P2 between the outer edge portions 108 and 128. Therefore, in the vicinity of the boundary between the plane P1 and the plane P2 in particular, a stronger flow path resistance can be exercised and the force of the fluid discharged from the biological tissue L can be weakened as compared with the case where the plane P1 and the plane P2 are the same flat plane.

### [Third Modification of First Embodiment]

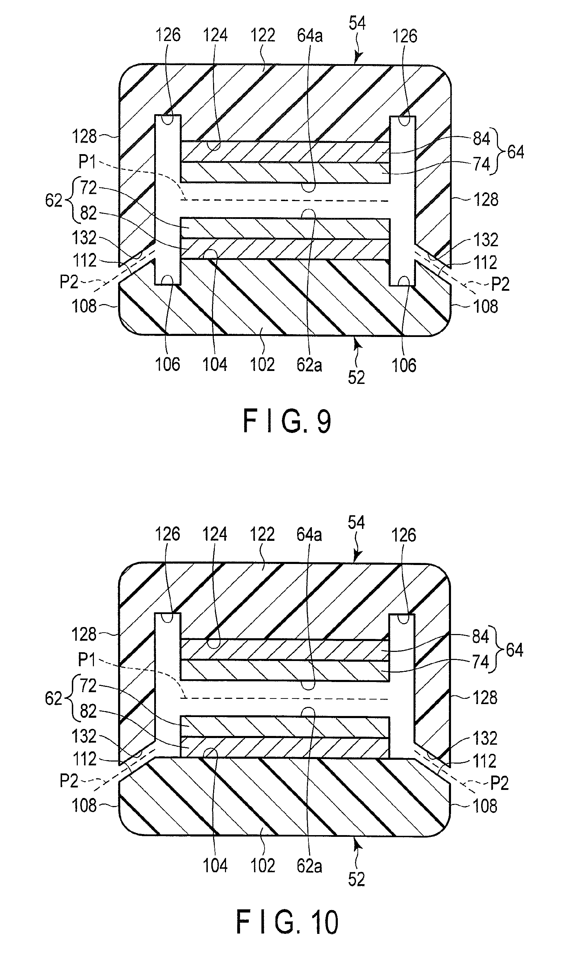

A third modification of the first embodiment will now be described with reference to FIG. 9.

As shown in FIG. 9, this modification is a modification of the second modification, and is an example where the inclined surfaces 112 and 132 are formed on the outer edge portions 108 and 128 as described in the first embodiment.

Since the outer edge portions 108 and 128 are formed as the inclined surfaces with respect to the surfaces (the holding surfaces) 62a and 64a of the electrodes 72 and 74, areas of the outer edge portions 108 and 128 can be increased as compared with the case where the outer edge portions 108 and 128 are parallel to the surfaces 62a and 64a of the electrodes 72 and 74. Therefore, the length from the inner side toward the outer side of each of the outer edge portions 108 and 128 (the width W of each of the inclined surfaces 112 and 132) can be increased without changing the width (the thickness t) of each of the outer edge portions 108 and 128. Therefore, the path from the outer edge of each of the energy discharge sections 62 and 64 to the outer edge of each of the outer edge portions 108 and 128 can be increased without changing the lateral width of each of the first and second jaws 52 and 54. Therefore, the spreading of heat can be efficiently suppressed without changing the thickness of each of the outer edge portions 108 and 128.

Further, when the configuration that the surrounding tissue S can be bent with respect to the biological tissue L as the treatment target is adopted, like the second modification, the flowing direction of the fluid discharged from the biological tissue L under the influence of the energy discharge sections 62 and 64 can be changed from the direction parallel to the virtual plane P1 between the surfaces 62a and 64a of the energy discharge sections 62 and 64 to the direction parallel to the virtual plane P2 between the outer edge portions 108 and 128. Therefore, in the vicinity of the boundary between the plane P1 and the plane P2, a strong flow path resistance can be exercised and the force of the fluid discharged from the biological tissue L can be weakened as compared with the case where the plane P1 and the plane P2 are the same flat plane.

### [Fourth Modification of First Embodiment]

A fourth modification of the first embodiment will now be described with reference to FIG. 10.

As shown in FIG. 10, this modification is an example where the groove 106 is removed from the first jaw 52 according to the third modification. That is, in this modification, although the outer edge portion 108 is present in the first jaw 52, the first jaw 52 does not have a barrier function. On the other hand, the second jaw 54 has a barrier function for preventing the spreading of heat with the use of the outer edge portion 128.

Since the outer edge portions 108 and 128 are formed as the inclined surfaces 112 and 132 with respect to the surfaces (the holding surfaces) 62a and 64a of the electrodes 72 and 74, areas of the outer edge portions 108 and 128 can be increased as compared with a case where the outer edge portions 108 and 128 are parallel to the surfaces 62a and 64a of the electrodes 72 and 74. Therefore, the length from the inner side toward the outer side of each of the outer edge portions 108 and 128 (the width W of each of the inclined surfaces 112 and 132) can be increased without changing the width (the thickness t) of each of the outer edge portions 108 and 128. Therefore, the path from the outer edge of each of the energy discharge sections 62 and 64 to the outer edge of each of the outer edge portions 108 and 128 can be increased without changing the lateral width of each of the first and second jaws 52 and 54. Therefore, the spreading of heat can be efficiently suppressed without changing the thickness of each of the outer edge portions 108 and 128.

Further, when the configuration that the surrounding tissue S can be bent with respect to the biological tissue L as the treatment target is adopted, like the second modification, the flowing direction of the fluid discharged from the biological tissue L under the influence of the energy discharge sections 62 and 64 can be changed from the direction parallel to the virtual plane P1 between the surfaces 62a and 64a of the energy discharge sections 62 and 64 to the direction parallel to the virtual plane P2 between the outer edge portions 108 and 128. Therefore, in the vicinity of the boundary between the plane P1 and the plane P2, a strong flow path resistance can be exercised and the force of the fluid discharged from the biological tissue L can be weakened as compared with the case where the plane P1 and the plane P2 are the same flat plane.

Furthermore, in this modification, the first jaw 52 can be formed to be relatively small. Accordingly, operability at the time of a treatment performed on the biological tissue, such as insertion of the first jaw 52 between biological tissues, can be improved.

### [Fifth Modification of First Embodiment]

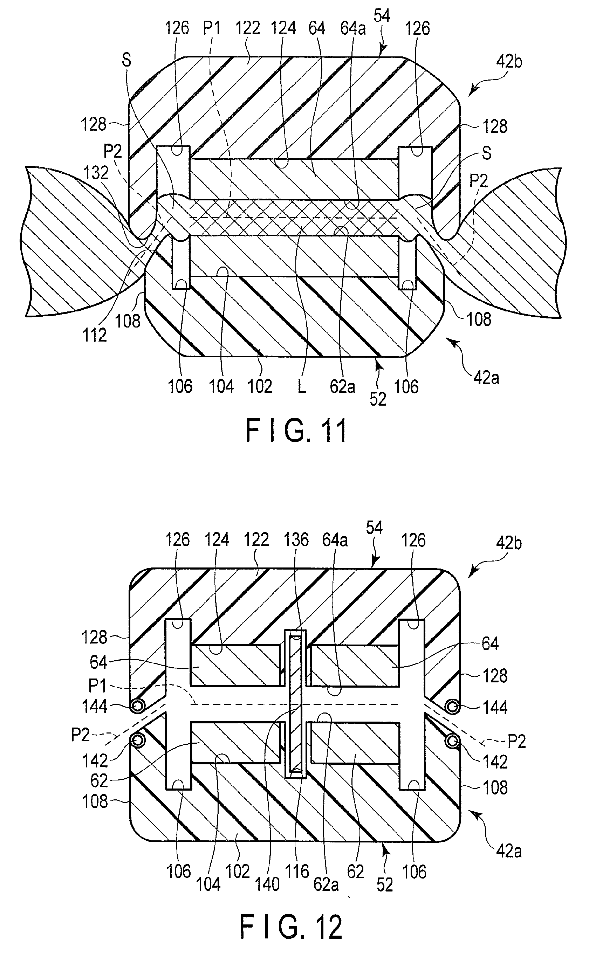

A fifth modification of the first embodiment will now be described with reference to FIG. 11.

As shown in FIG. 11, a lateral width of a lateral cross section of the first jaw 52 is formed to be smaller than a lateral width of a lateral cross section of the second jaw 54. Therefore, as shown in FIG. 11, the outer side of the first outer edge portion 108 of the first jaw 52 can be arranged on the inner side of the second outer edge portion 128 of the second jaw 54.

The inclined surface portion 112 of the first jaw 52 is formed on the outer side of the first outer edge portion 108, and the inclined surface portion 132 of the second jaw 54 is formed on the inner side of the second outer edge portion 128.

As shown in FIG. 11, when the biological tissue is held between the first and second treatment sections 42a and 42b, the biological tissue L as the treatment target is present on the virtual plane P1 formed by collecting midpoints between the surfaces 62a and 64a of the electrodes 72 and 74. Further, the surrounding tissue S of the biological tissue L as the treatment target is bent from the grooves 106 and 126 toward the outer edge portions 108 and 128, and is present on the virtual plane P2 formed by collecting midpoints between the inclined surface portions 112 and 132 by the outer side of the outer edge portion 108 of the first jaw 52 and the inner side of the outer edge portion 128 of the second jaw 54. A shear force that does not lead to cutting can act on the surrounding tissue S. Therefore, the surrounding tissue S can be bent with respect to the biological tissue L as the treatment target, and the spreading of heat to the surrounding tissue S of the biological tissue L as the treatment target can be effectively suppressed.

It is to be noted that, in this modification, the description has been given as to the example where the outer side of the first outer edge portion 108 of the first jaw 52 is arranged on the inner side of the second outer edge portion 128 of the second jaw 54, but the inner side of the first outer edge portion 108 of the first jaw 52 may be arranged on the outer side of the second outer edge portion 128 of the second jaw 54.

### [Sixth Modification of First Embodiment]

A sixth modification of the first embodiment will now be described with reference to FIG. 12. This modification is a modification of the first embodiment including the foregoing modifications.

As shown in FIG. 12, cutter guide grooves 116 and 136 are formed in the first and second jaws 52 and 54, respectively. A cutter 140 can be inserted into or removed from the cutter guide grooves 116 and 136. The cutter 140 is coupled with a cutter moving lever 46b of the operating section 46 shown in FIG. 1 through a non-illustrated rod. Therefore, when the cutter moving lever 46b is operated, the cutter 140 can be guided within a predetermined range along an axial direction of the inserting section 44. That is, the cutter 140 can be moved between a state that a tip of the cutter 140 is arranged at a position between the first and second jaws 52 and 54 and a state that the tip of the cutter 140 is retracted in the inserting section 44 from the position between the first and second jaws 52 and 54. Therefore, after giving a treatment by applying the energy to the biological tissue L as the treatment target from the energy discharge sections 62 and 64, when the cutter moving lever 46b is operated to move the tip of the cutter 140 to the distal end side of the first and second jaws 52 and 54 from the proximal end side of the first and second jaws 52 and 54, the biological tissue L as the treatment target can be cut.

Here, as shown in FIG. 5A and FIG. 5B, since the outer edge portions 108 and 128 can hold the surrounding tissue S in a bent state, the surrounding tissue S can be firmly held by the outer edge portions 108 and 128. Therefore, when the cutter 140 has been moved, it is hard for the surrounding tissue S between the outer edge portions 108 and 128 to slip, and the biological tissue L that has been subjected to a treatment can be assuredly cut.

It is to be noted that the cutter guide grooves 116 and 136 have a function of receiving the fluid generated from the biological tissue, like the grooves 106 and 126.

Further, cooling ducts (cooling sections) 142 and 144 are formed in the inclined surfaces 112 and 132 of the outer edge portions 108 and 128. A cooling medium can be circulated in the cooling ducts 142 and 144. Therefore, for example, the biological tissue L is held between the holding surfaces 62a and 64a, the surrounding tissue S is held between the inclined surfaces 112 and 132, the energy is discharged from the energy discharge sections 62 and 64, and the cooling medium is circulated through the cooling ducts 142 and 144 to cool the surrounding tissue S, whereby the spreading of heat can be further effectively suppressed.

It is to be noted that, as shown in FIG. 12, the description has been given as to the example where the cooling ducts 142 and 144 are arranged on the outer edges of the inclined surfaces 112 and 132 of the outer edge portions 108 and 128 in this modification, but they may be arranged at centers of the inclined surfaces 112 and 132 of the outer edge portions 108 and 128 in the width direction or on inner edge portions of the same, respectively. Moreover, the cooling ducts 142 and 144 do not have to be formed when the energy discharge section is not provided on the holding surface 62a, like the first modification (see FIG. 7).

Additionally, all the surrounding tissue S pressed by the inclined surfaces 112 and 132 may be cooled by arranging plates having excellent thermal conductivity on the inclined surfaces 112 and 132, and the heat of the cooling ducts 142 and 144 is therefore transferred to the plates.

Since other structures or functions are the same as the structures and the functions described in the first embodiment, a description thereof will be omitted here.

### [Second Embodiment]

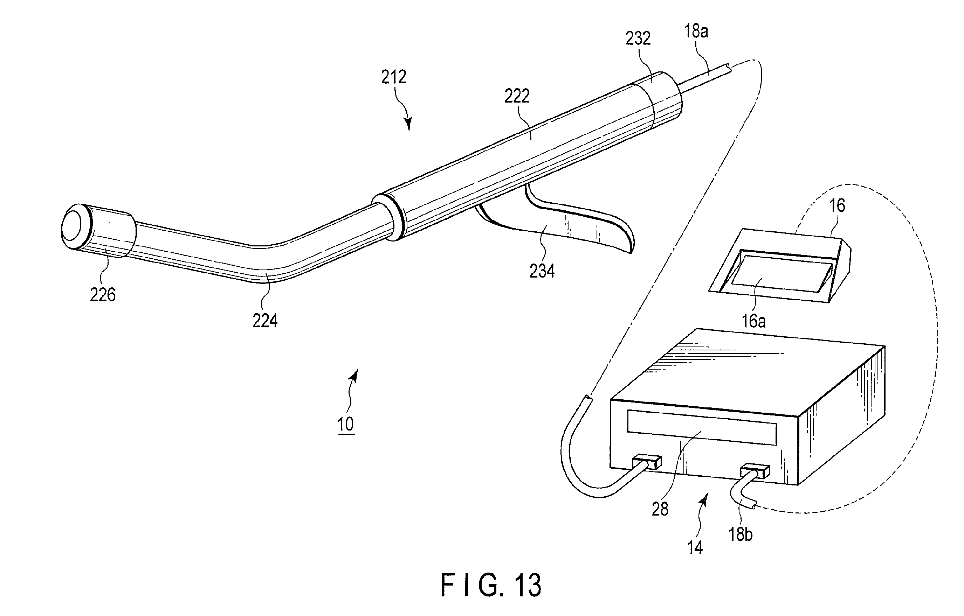

A second embodiment will now be described with reference to FIG. 13 to FIG. 15A. This embodiment is a modification of the first embodiment including the respective modifications, and like reference numerals denote the same members or members having the same functions as the members described in the first embodiment as much as possible to omit a repeated description.

Here, as an energy treatment device, a description will be given as to an example of a circular type surgical device (the energy treatment device) 212 used for giving a treatment through an abdominal wall or outside the abdominal wall. In this embodiment, although a bipolar type surgical device 212 will be described, the treatment device may be formed as a monopolar type energy treatment device by using a counter electrode plate R shown in FIG. 6B.

As shown in FIG. 13, a treatment system 10 using energy has a treatment device (the energy treatment device) 212, an energy source 14 that gives energy to the treatment device 212, and a foot switch 16.

The surgical device 212 includes a handle (an operating section) 222, a shaft (an inserting section) 224, and an openable/closable treatment section 226. The energy source 14 is connected to the handle 222 through a cable 18a.

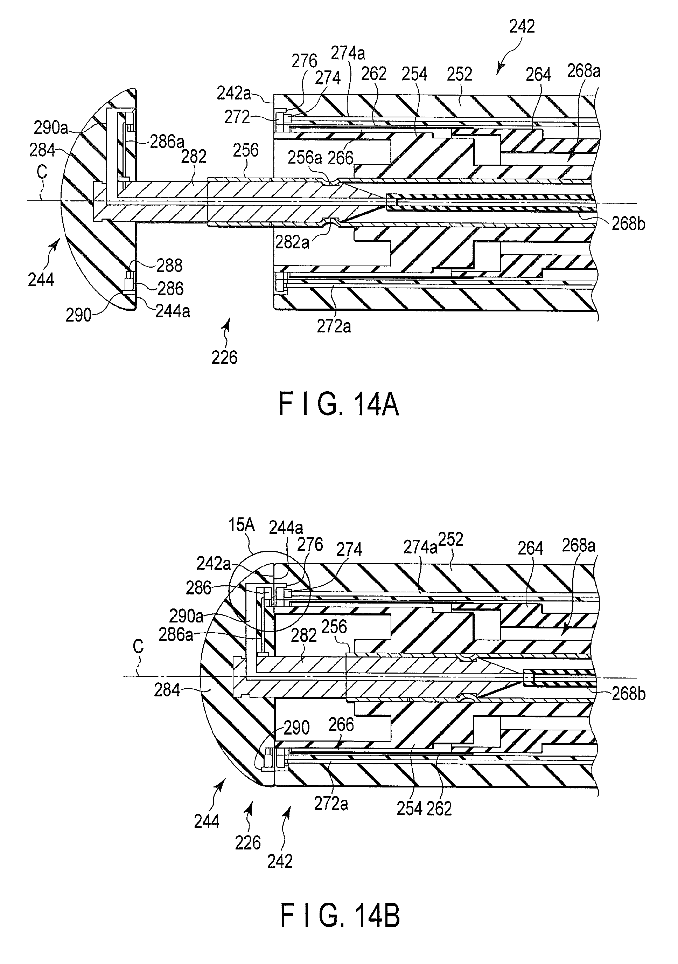

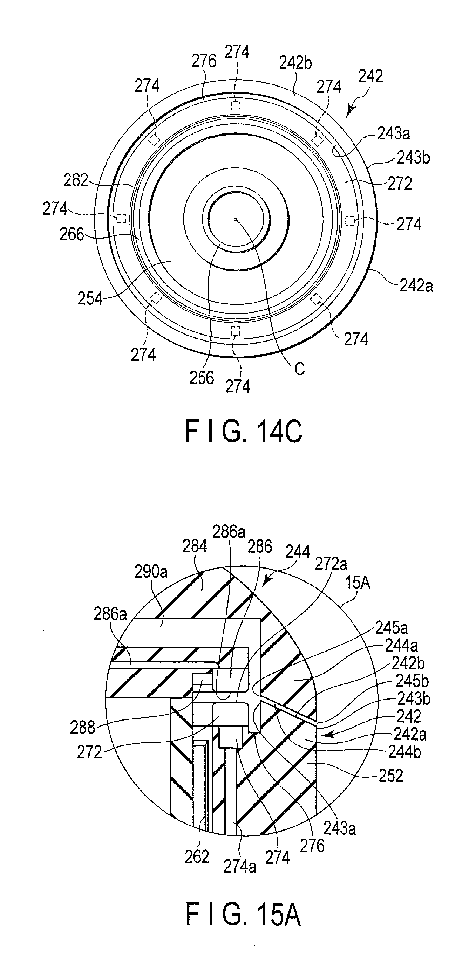

A holding section opening/closing knob 232 and a cutter driving lever 234 are arranged on the handle 222. The holding section opening/closing knob 232 is rotatable with respect to the handle 222. A later-described separation side treatment section (a separation side grip section) 244 of the treatment section 226 is separated from a main body side treatment section (a main body side grip section) 242 when this holding section opening/closing knob 232 is rotated in, e.g., a clockwise direction with respect to the handle 222 (see FIG. 14A), and the separation side treatment section 244 moves closer to the main body side treatment section 242 when the holding section opening/closing knob 232 is rotated in a counterclockwise direction (see FIG. 14B).

As shown in FIG. 13, the shaft 224 is formed into a cylindrical shape. This shaft 224 is appropriately curved in consideration of insertability into a biological tissue L. Forming the shaft 224 straight is also preferable.

The treatment section 226 is arranged at a distal end of the shaft 224. As shown in FIG. 14A and FIG. 14B, the treatment section 226 includes a main body side treatment section (a first holding member, a first jaw) 242 formed at the distal end of the shaft 224 and a separation side treatment section (a second holding member, a second jaw) 244 that can be attached to or detached from this main body side treatment section 242. In a state that the separation side treatment section 244 is closed with respect to the main body side treatment section 242, outer edge portions 242a and 244a of the main body side treatment section 242 and the separation side treatment section 244 move closer to each other in an opposed state or abut on each other. Therefore, the circular outer edge portion 242a faces the circular outer edge portion 244a and forms a holding section (the second holding section) that holds a surrounding tissue S of a biological tissue L as a treatment target.

As shown in FIG. 15A, in this embodiment, the outer edge portions 242a and 244a are inclined with respect to a central axis C (see FIG. 14A and FIG. 14B) of the main body side treatment section 242 and the separation side treatment section 244 and a direction orthogonal to the central axis C.

The outer edge portion 242a itself of the main body side treatment section 242 has an end portion (the uppermost end) 243a on an inner side thereof (a side close to a high-frequency electrode 272) provided above an end portion 243b on an outer side thereof (a side apart from the first high-frequency electrode 272) in this embodiment, and the end portion 243a on the inner side is smoothly continuous with the end portion 243b on the outer side. Therefore, an inclined surface 242b is formed on the outer edge portion 242a.

The outer edge portion 244a itself of the separation side treatment section 244 has an end portion (the uppermost end) 245a on an inner side thereof (a side close to a second high-frequency electrode 286) provided above an end portion 245b on an outer side thereof (a side apart from the second high-frequency electrode 286) in this embodiment, and the inner end portion 245a is smoothly continuous with the outer end portion 245b. Therefore, an inclined surface 244b is formed on the outer edge portion 244a.

As shown in FIG. 14A and FIG. 14B, the main body side treatment section 242 includes a cylindrical body 252, a frame 254, and an energization pipe 256. The cylindrical body 252 and the frame 254 have electrical insulation properties. The cylindrical body 252 is coupled with the distal end of the shaft 224. The frame 254 is arranged while being fixed to the cylindrical body 252.

The frame 254 has opening in a central axis thereof. The energization pipe 256 is arranged in the opened central axis C of this frame 254 so that it can move within a predetermined range along the central axis C of the frame 254. As shown in FIG. 14A and FIG. 14B, when the holding section opening/closing knob 232 is rotated, this energization pipe 256 can move within the predetermined range by a function of, e.g., a ball screw (not shown). A protrusion 256a that projects inward along a radial direction is formed on this energization pipe 256 so that a later-described connecting portion 282a of the energization shaft 282 in the separation side treatment section 244 can be engaged or disengaged.

As shown in FIG. 14A to FIG. 14C, a cutter guide groove (a space) 266 is formed between the cylindrical body 252 and the frame 254. A cylindrical cutter 262 is arranged in the cutter guide groove 266. A proximal end portion of this cutter 262 is connected to a distal end portion of a cutter pusher 264 arranged on the inner side of the shaft 224. The cutter 262 is fixed on an outer peripheral surface of the cutter pusher 264. Although not shown, a proximal end portion of the cutter pusher 264 is connected to the cutter driving lever 264 of the handle 222. Therefore, when the cutter driving lever 234 of the handle 222 is operated, the cutter 262 moves through the cutter pusher 264.

A first fluid ventilation path (a fluid path) 268a is formed between the cutter pusher 264 and the frame 254. Further, a fluid discharge opening (not shown) from which the fluid flowing through the first fluid ventilation path 268a is discharged to the outside is formed in the shaft 224 or the handle 222.

As shown in FIG. 14A and FIG. 14B, as output members or energy discharge sections, the first high-frequency electrode 272 and heating members 274 are arranged at a distal end of the cylindrical body 252.

The first high-frequency electrode 272 is arranged on an outer side of the cutter guide groove 266 having the cutter 262 arranged therein. The first high-frequency electrode 272 is formed into a circular shape like the cutter guide groove 266. A distal end of a first energization line 272a is fixed to the first high-frequency electrode 272. The first energization line 272a is connected to a cable 18a through the main body side treatment section 242, the shaft 224, and the handle 222.

As shown in FIG. 14A to FIG. 14C, the heating members 274 are fixed on a back surface of the first high-frequency electrode 272 at appropriate intervals. A tip of a heater energization line 274a is fixed to each heating member 274. The heater energization line 274a is connected to the cable 18a through the main body side treatment section 242, the shaft 224, and the handle 222.

It is to be noted that using one or more tabular heaters as the heating members 274 is also preferable.

A fluid discharge groove 276 is annularly formed on the outer side of the first high-frequency electrode 272. The fluid discharge groove 276 is formed to communicate with the first fluid ventilation path 268a. The inclined surface (a tissue contact surface) 242b of the outer edge portion 242a is formed on the outer side of the fluid discharge groove 276 at a position protruding from the surface of the first high-frequency electrode 272. That is, the inclined surface 242b of the outer edge portion 242a of the main body side treatment section 242 is closer to a later-described head section 284 of the separation side treatment section 244 than the surface of the first high-frequency electrode 272. Therefore, the end portion (the uppermost end) 243a on the inner side of the outer edge portion 242a (the side close to the first high-frequency electrode 272) functions as a barrier section (a dam) that prevents a fluid such as vapor from escaping to the outer side of the fluid discharge groove 276.

On the other hand, the separation side treatment section 244 includes an energization shaft 282 having the connecting portion 282a and the head section 284. The energization shaft 282 has a circular cross section, one end formed into a tapered shape, and the other end fixed to the head section 284. The connecting portion 282a is formed into a concave groove shape that can be engaged with the protrusion 256a of the energization pipe 256. An outer surface of the energization shaft 282 except for the connecting portion 282a is insulated by a coating or the like.

The second high-frequency electrode 286 is arranged in the head section 284 to face the first high-frequency electrode 272 of the main body side treatment section 242. One end of a second energization line 286a is fixed to the second high-frequency electrode 286. The other end of the second energization line 286a is electrically connected to the energization shaft 282.

The high-frequency electrodes 272 and 286 face each other and are used as holding surfaces (a first holding section) 273 and 286 for the biological tissue L as the treatment target. Therefore, when high-frequency energy is given to the electrodes 272 and 286 in a state that the biological tissue L is held between the holding surfaces 273 and 287 of the electrodes 272 and 286, the biological tissue L can be denatured by thermal energy that has heated the biological tissue. Further, the electrode 272 is made of a material having excellent thermal conductivity. Therefore, when the heating member 274 is heated, the heat (thermal energy) is transferred to the electrode 272, and the heat (the thermal energy) can be further transferred to the biological tissue L that is in contact with the holding surface 273 of the electrode 272. Therefore, the holding surfaces 273 and 287 also function as treatment surfaces for the biological tissue L. It is to be noted that the heating members are not arranged on the back surface of the electrode 286 in this embodiment, but the electrode 286 may be made of a material having excellent thermal conductivity, the heating members may be arranged on the back surface of the electrode 286, and the heat generated by the heating members may be transferred to the electrode 286.

A cutter receiving portion 288 is annularly formed on the inner side of the second high-frequency electrode 286 arranged in the head section 284 so that a blade at a tip of the cutter 262 can be received. On the other hand, a fluid discharge groove 290 is annularly formed on the outer side of the second high-frequency electrode 286. On the outer side of the fluid discharge groove 290, an inclined surface (a tissue contact surface) 244b of the outer edge portion 244a is formed at a position protruding from the surface of the second high-frequency electrode 286. That is, the outer edge portion 244a of the separation side treatment section 244 is arranged to be closer to the main body side treatment section 242 than the surface of the second high-frequency electrode 286. Therefore, the end portion 245a on the inner side (the side close to the second high-frequency electrode 286) of the outer edge portion 244a functions as a barrier section (a dam) that prevents a fluid such as vapor from escaping to the outside from the fluid discharge groove 290.

Furthermore, the fluid discharge groove 290 is configured to communicate with a fluid discharge path 290a of the head section 284 and the energization shaft 282. The fluid discharge path 290a communicates with a second fluid ventilation path (a fluid path) 268b of the energization pipe 256. A fluid discharge opening (not shown) from which the fluid flowing through the second fluid ventilation path 268b is discharged to the outside is formed in the shaft 204 or the handle 202.

It is to be noted that the energization pipe 256 is connected to the cable 18a through the shaft 224 and the handle 222. Therefore, when the connecting portion 282a of the energization shaft 282 in the separation side treatment section 244 is engaged with the protrusion 256a of the energization pipe 256, the second high-frequency electrode 286 is electrically connected to the energization pipe 256.

A function of the therapeutic treatment system 10 according to this embodiment will now be described.

An operator operates the display section 28 (see FIG. 2 and FIG. 13) of the energy source 14 in advance to set output conditions of the therapeutic treatment system 10. Specifically, set power Pset [W] of a high-frequency energy output, a set temperature Tset [°C] of a thermal energy output, threshold values Z1 and Z2 of impedance Z of the biological tissue L, and so on are set in advance.

As shown in FIG. 14B, in a state that the main body side treatment section 242 is closed with respect to the separation side treatment section 244, the treatment section 226 and the shaft 224 of the surgical treatment device 212 are inserted into, e.g., an abdominal cavity through an abdominal wall. The main body side treatment section 242 and the separation side treatment section 244 of the surgical treatment device 212 are arranged to face a biological tissue as a treatment target.

To grip the biological tissue as the treatment target with the use of the main body side treatment section 242 and the separation side treatment section 244, the grip section opening/closing knob 232 of the handle 222 is operated. At this time, the knob 232 is turned in, e.g., the clockwise direction with respect to the handle 222. Then, as shown in FIG. 14A, the energization pipe 256 is moved to the distal end portion side with respect to the frame 254 of the shaft 224. Therefore, a space is formed between the main body side treatment section 242 and the separation side treatment section 244, and the separation side treatment section 244 can be separated from the main body side treatment section 242.

Additionally, the biological tissue L to be subjected to a treatment is arranged between the first high-frequency electrode 272 of the main body side treatment section 242 and the second high-frequency electrode 286 of the separation side treatment section 244. The energization shaft 282 of the separation side treatment section 244 is inserted into the energization pipe 256 of the main body side treatment section 242. In this state, the grip section opening/closing knob 232 of the handle 222 is turned in, e.g., the counterclockwise direction. Therefore, the separation side treatment section 244 is closed with respect to the main body side treatment section 242. In this manner, the biological tissue L as the treatment target is held between the main body side treatment section 242 and the separation side treatment section 244.

When the biological tissue is held between the holding surfaces 273 and 287 of the electrodes 272 and 286 and between the inclined surfaces 242b and 244b of the outer edge portions 242b and 244a, a virtual plane formed by collecting midpoints between the holding surfaces 273 and 287 of the electrodes 272 and 286 crosses a virtual plane formed by collecting midpoints between the inclined surfaces 242b and 244b of the outer edge portions 242a and 244a. Therefore, the biological tissue between the outer edge portions 242a and 244a (a surrounding tissue of the biological tissue as the treatment target) can be held while being bent with respect to the biological tissue between the holding surfaces 273 and 287 of the electrodes 272 and 286 (the biological tissue as the treatment target).

As compared with a case where an angle of each of the outer edge portions 242a and 244a is orthogonal to the central axis C, a width of each of the inclined surfaces 242b and 244b of the outer edge portions 242a and 244a according to this embodiment is increased in accordance with the angle. Therefore, when the inclined surfaces 242b and 244b are formed on the outer edge portions 242a and 244a, a contact area of the biological tissue as the treatment target with respect to the surrounding tissue can be increased as compared with a case where an angle of each of the inclined surfaces 242b and 244b of the outer edge portions 242a and 244a is orthogonal to the central axis C.

In this state, a pedal 216a of a foot switch 216 is operated, and energy is supplied to the first high-frequency electrode 272 and the second high-frequency electrode 286 from the energy source 14 through the cable 18a, respectively. Therefore, the biological tissue L between the first high-frequency electrode 272 of the main body side treatment section 242 and the second high-frequency electrode 286 of the separation side treatment section 244 is heated by Joule heat.

As the biological tissue is heated, a fluid (a liquid (blood) and/or a gas (water vapor)) is discharged from the biological tissue. At this time, the fluid discharged from the biological tissue L is allowed to flow into the cutter guide groove 266 and the fluid discharge groove 276 of the main body side treatment section 242, and also into the fluid discharge groove 290 of the separation side treatment section 244. Further, for example, the fluid that has flowed into the cutter guide groove 266 and the fluid discharge groove 276 of the main body side treatment section 242 is sucked into the shaft 224 from the cutter guide groove 266 through the first fluid ventilation path 268a. Furthermore, for example, the fluid that has flowed into the fluid discharge groove 290 of the separation side treatment section 244 is sucked into the shaft 224 from the fluid discharge path 290a of the head section 284 and the energization shaft 282 through the second fluid ventilation path 268b of the energization pipe 256.

Moreover, the inflow of the fluid is continued while the fluid is being discharged from the biological tissue L. Therefore, the fluid discharged from the biological tissue L in a state that a temperature is raised can be prevented from spreading heat and also prevented from affecting a part that is not a treatment target.

Additionally, since the surrounding tissue is bent with respect to the biological tissue as the treatment target, a strong flow path resistance can be exercised, and the force of the fluid discharged from the biological tissue L can be weakened. Further, as regards areas of the inclined surfaces 242b and 244b of the outer edge portions 242a and 244a, since the inclined surfaces 242b and 244b are formed to be inclined with respect to the plane orthogonal to the central axis C, contact areas relative to the surrounding tissue can be increased as compared with a case where the inclined surfaces 242b and 244b are formed as planes orthogonal to the central axis C. Furthermore, when a shear force that does not lead to cutting is enabled to act on the surrounding tissue S, the surrounding tissue can be further assuredly held. Therefore, the spreading of heat relative to the surrounding tissue of the biological tissue as the treatment target can be effectively suppressed.

When the impedance Z has been determined to be higher than the threshold value Z1, a signal is transmitted from the control section 22 to a heating element drive circuit 26. Moreover, the heating element drive circuit 26 supplies electric power to the heating members 274 in such a manner that a temperature of the heating members 274 becomes the preset temperature Tset [°C], e.g., 100[°C] to 300[°C]. Therefore, as to the biological tissue gripped between the electrodes 272 and 286 of the main body side treatment section 242 and the separation side treatment section 244, heat is transferred to the first high-frequency electrode 272 by heat conduction from the heating members 274, and the biological tissue is coagulated by this heat from the surface side toward the inside of the biological tissue appressed against the first high-frequency electrode 272.

Then, the control section 22 determines whether the impedance Z of the biological tissue monitored by the high-frequency energy output circuit 24 has become greater than or equal to the preset threshold value Z2. When the impedance Z has been determined to be smaller than the threshold value Z2, supply of the energy to the heating members 274 is continued. On the other hand, when the impedance Z has been determined to be greater than or equal to the threshold value Z2, the control section 22 generates a buzzer from a speaker 30 and stops output of the high-frequency energy and the thermal energy. Therefore, the treatment for the biological tissue using the therapeutic treatment system 10 is terminated.

As described above, the biological tissue is continuously denatured (in a substantially annular state) by the first and second high-frequency electrodes 272 and 286 and the heating member 274.

Moreover, when the cutter driving lever 234 of the handle 222 is operated, the cutter 262 projects from the cutter guide groove 266 of the main body side treatment section 242 and moves toward the cutter receiving portion 288 in the separation side treatment section 244. Since the blade of the cutter 262 is at the tip thereof, the biological tissue subjected to the treatment is cut into an arc shape, a circular shape, or the like.

At this time, since the surrounding tissue is firmly held by the inclined surfaces 242b and 244b, it is hard for the surrounding tissue between the inclined surfaces 242b and 244b to slip with respect to the inclined surfaces 242b and 244b, and the biological tissue can be easily cut by the cutter 262.

As described above, according to this embodiment, the following effect can be provided.

The first high-frequency electrode 272 and the heating members 274 can be annularly arranged in the main body side treatment section 242 and the second high-frequency electrode 286 can be annularly arranged in the separation side treatment section 244 to give a treatment. Therefore, the biological tissue L between the main body side treatment section 242 and the separation side treatment section 244 can be substantially annularly treated.

When the biological tissue is held between the holding surfaces 273 and 287 of the electrodes 272 and 286 and between the inclined surfaces 242b and 244b of the outer edge portions 242a and 244a, the virtual plane formed by collecting midpoints between the holding surfaces 273 and 287 of the electrodes 272 and 286 crosses the virtual plane formed by collecting midpoints between the inclined surfaces 242b and 244b of the outer edge portions 242a and 244a. Therefore, the biological tissue between the inclined surfaces 242b and 244b of the outer edge portions 242a and 244a (the surrounding tissue of the biological tissue as the treatment target) can be held in a bent state with respect to the biological tissue between the holding surfaces 273 and 287 of the electrodes 272 and 286 (the biological tissue as the treatment target). As described above, when the biological tissue is held in such a manner that its surrounding tissue is bent with respect to the biological tissue as the treatment target, a strong flow path resistance can be exercised near a boundary between the biological tissue as the treatment target and the surrounding tissue with respect to the fluid discharged from the biological tissue, and the force of the fluid discharged from the biological tissue L can be weakened.

The width of the outer edge portion 242a according to this embodiment is increased in accordance with the angle of the inclined surface 242b of the outer edge portion 242a as compared with the case where the angle is orthogonal to the central axis C. Therefore, if the inclined surface 242b is formed on the outer edge portion 242a, the contact area of the biological tissue as the treatment target with respect to the surrounding tissue can be increased as compared with a case where the angle of the inclined surface 244b of the outer edge portion 242a is orthogonal to the central axis C. Therefore, the spreading of heat of the biological tissue as the treatment target relative to the surrounding tissue can be effectively suppressed.

Further, in the second embodiment, although the description has been given as to the case where the high-frequency energy is provided by the electrodes 272 and 286 and the thermal energy is provided by the heating members 274, the high-frequency energy provided by the electrodes 272 and 286 or the thermal energy provided by the heating members 274 alone may be used as the energy.

### [First Modification of Second Embodiment]

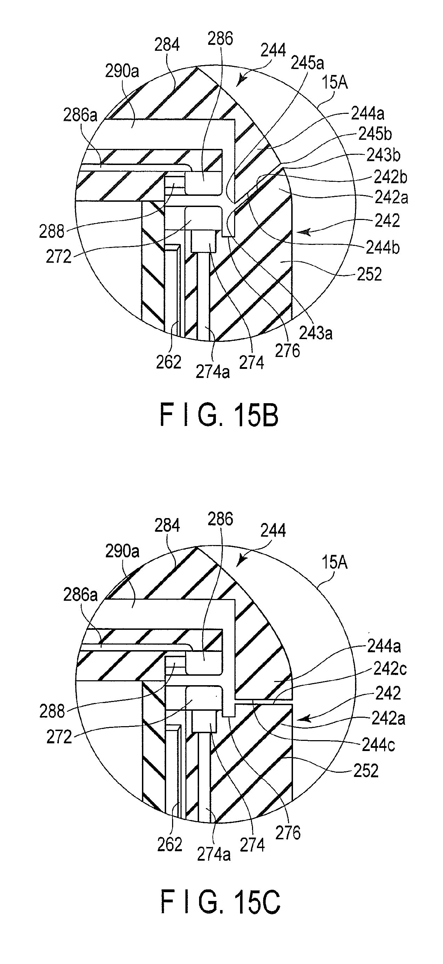

A first modification of the second embodiment will now be described with reference to FIG. 15B. It is to be noted that like reference numerals denote the same members or members having the same functions as the members described in the second embodiment as much as possible to omit a detailed description thereof.

As shown in FIG. 15B, this modification is an example in which inclining directions of the inclined surfaces 242b and 244b of the outer edge portions 242a and 244a are changed with respect to the second embodiment (see FIG. 15A).

In this modification, the outer edge portion 242a itself has the end portion (the lowermost end) 243a on the inner side thereof (the side close to the first high-frequency electrode 272) provided below the end portion 243b on the outer side thereof (the side apart from the first high-frequency electrode 272), and the end portion 243a on the inner side is smoothly continuous with the end portion 243b on the outer side. Therefore, the inclined surface 242b is formed on the outer edge portion 242a.

In this modification, the outer edge portion 244a itself has the end portion (the lowermost end) 245a on the inner side thereof (the side close to the second high-frequency electrode 286) provided below the end portion 245b on the outer side thereof (the side apart from the second high-frequency electrode 286), and the end portion 245a on the inner side is smoothly continuous with the end portion 245b on the outer side. Therefore, the inclined surface 244b is formed on the outer edge portion 244a.

As described in the second embodiment, when the biological tissue is held between the holding surfaces 273 and 287 of the electrodes 272 and 286 and between the inclined surfaces 242b and 244b of the outer edge portions 242a and 244a, the biological tissue between the inclined surfaces 272b and 244b of the outer edge portions 242a and 244a (the surrounding tissue of the biological tissue as the treatment target) can be held in a bent state with respect to the biological tissue between the holding surfaces 273 and 287 of the electrodes 272 and 286 (the biological tissue as the treatment target). Furthermore, the contact area of the surrounding tissue can be increased as compared with the case of forming flat surfaces orthogonal to the central axis C.

### [Second Modification of Second Embodiment]

A second modification of the second embodiment will now be described with reference to FIG. 15C.

As shown in FIG. 15C, this modification is an example where the virtual plane formed by collecting midpoints between the holding surfaces 273 and 287 of the electrodes 272 and 286 is parallel to the virtual plane formed by collecting midpoints between the flat surfaces 242c and 244c of the outer edge portions 242a and 244a, but these planes are arranged at different positions.

Although flat surfaces 242c and 244c parallel to the direction orthogonal to the central axis C (see FIG. 14A and FIG. 14B) of the main body side treatment section 242 and the separation side treatment section 244 are formed on the outer edge portions 242a and 244a. Positions of the midpoints between the flat surfaces 242c and 244c of the outer edge portions 242a and 244a are positions closer to the lower end of the fluid discharge groove 276 than the midpoints between the holding surfaces 273 and 287 of the electrodes 272 and 286.

Therefore, when the biological tissue is held between the holding surfaces 273 and 287 of the electrodes 272 and 286 and between the flat surfaces 242c and 244c of the outer edge portions 242a and 244a, the biological tissue between the flat surfaces 242c and 244c of the outer edge portions 242a and 244a can be held in a bent state with respect to the biological tissue between the holding surfaces 273 and 287 of the electrodes 272 and 286. Furthermore, since a length from the virtual plane formed by collecting the midpoints between the holding surfaces 273 and 287 of the electrodes 272 and 286 to the virtual plane formed by collecting the midpoints between the flat surfaces 242c and 244c of the outer edge portions 242a and 244a can be increased, the force of the fluid discharged from the biological tissue L can be weakened, and occurrence of the spreading of heat relative to the surrounding tissue can be suppressed.

### [Third Modification of Second Embodiment]

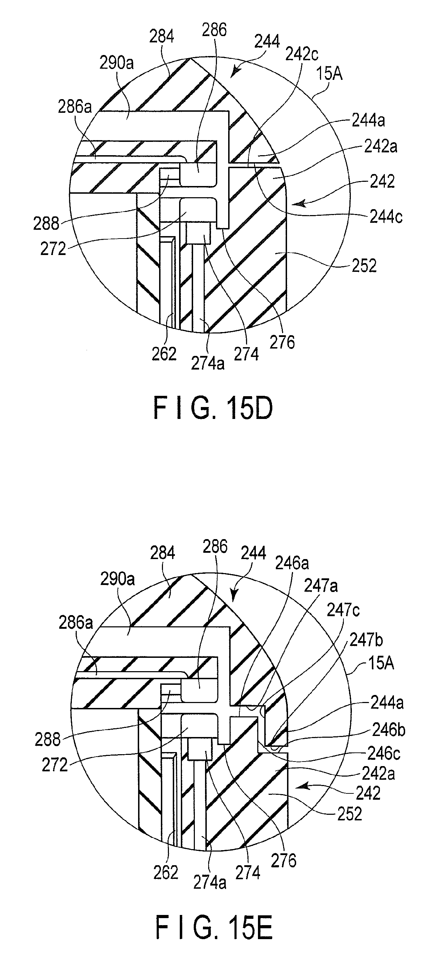

A third modification of the second embodiment will now be described with reference to FIG. 15D.

As shown in FIG. 15D, this modification is an example in which positions of the outer edge portions 242a and 244a are changed with respect to the second modification (see FIG. 15C).

The outer edge portions 242a and 244a are formed parallel to the direction orthogonal to the central axis C (see FIG. 14A and FIG. 14B) of the main body side treatment section 242 and the separation side treatment section 244. Moreover, positions of the midpoints between the flat surfaces 242c and 244c of the outer edge portions 242a and 244a are positions closer to the upper end of the fluid discharge groove 290 than the midpoints between the holding surfaces 273 and 287 of the electrodes 272 and 286.

Therefore, when the biological tissue is held between the holding surfaces 273 and 287 of the electrodes 272 and 286 and between the flat surfaces 242c and 244c of the outer edge portions 242a and 244a, the biological tissue between the flat surfaces 242c and 244c can be held in a bent state with respect to the biological tissue between the holding surfaces 273 and 287 of the electrodes 272 and 286.

### [Fourth Modification of Second Embodiment]

A fourth modification of the second embodiment will now be described with reference to FIG. 15E.

As shown in FIG. 15E, this modification is an example where the first outer edge portion 242a is arranged on the inner side of the second outer edge portion 244a and the biological tissue is bent between the outer edge portions 242a and 244a.

The outer edge portion 242a of the main body side treatment section 242 has an inner holding surface 246a, an outer holding surface 246b, and a coupling surface 246c. The coupling surface 246c is formed to couple the inner holding surface 246a with the outer holding surface 246b. The inner holding surface 246a and the outer holding surface 246b are formed to be substantially parallel to each other. The outer edge portion 244a of the separation side treatment section 244 has an inner holding surface 247a, an outer holding surface 247b, and a coupling surface 247c. The coupling surface 247c is formed to couple the inner holding surface 247a with the outer holding surface 247b. The inner holding surface 247a and the outer holding surface 247b are formed to be substantially parallel to each other. Further, the coupling surfaces 246c and 247c are formed as, e.g., surfaces substantially parallel to the central axis C.

Therefore, when the biological tissue is held between the holding surfaces 273 and 287 of the electrodes 272 and 286 and between the outer edge portions 242a and 244a, the biological tissue between the outer edge portions 242a and 244a can be held in a bent state with respect to the biological tissue between the holding surfaces 273 and 287 of the electrodes 272 and 286. At this time, since a shear force that does not lead to cutting can act on the surrounding tissue S, the surrounding tissue can be further firmly held.

It is to be noted that one of the holding surfaces 273 and 287 may be used as an energy discharge section and the other of the same may be configured not to discharge energy, like the first modification of the first embodiment (see FIG.7).

In this embodiment, likewise, bending the surrounding tissue with respect to the biological tissue as the treatment target enables increasing a length from the biological tissue as the treatment target to the outer edge of each of the treatment sections 242 and 244. Therefore, a direction of the surrounding tissue can be changed, and a contact area of each of the outer edge portions 242a and 244a can be increased. Therefore, occurrence of the spreading of heat relative to the surrounding tissue can be effectively suppressed.

It is to be noted that, in this embodiment, changing the length of each of the treatment sections 242 and 244 to the central axis C like the fifth modification of the first embodiment (see FIG. 11), i.e., forming the outer edge portions 242a and 244a with different outer diameters enables bending the surrounding tissue. Either the outer edge portion 242a or 244a may be formed with an increased outer diameter.

Although the several embodiments have been specifically described above with reference to the drawings, the present invention is not restricted to the foregoing embodiments, and it includes all embodiments carried out without departing from the gist thereof.

## Claims

1. A surgical device comprising a treatment section, configured to apply energy to a biological tissue as a treatment target, and treat the biological tissue as the treatment target, wherein the treatment section includes:
first and second jaws which are relatively openable/closable to each other to enable holding and releasing the biological tissue including the treatment target and its surrounding tissue;
a pair of mutually opposed first holding sections which are provided respectively on inner sides of outer edges of the first and second jaws, and configured to hold the biological tissue as the treatment target;
an energy discharge section which is provided on at least one of the pair of first holding sections, and configured to give energy for a treatment to the biological tissue as the treatment target; and
a pair of second holding sections which are provided respectively between the first holding sections and the outer edges of the first and second jaws, and configured to hold the surrounding tissue of the biological tissue as the treatment target while bending the surrounding tissue with respect to the biological tissue as the treatment target.

2. The surgical device according to claim 1, wherein the second holding sections are configured to hold the surrounding tissue in a pressed state, when the first and second jaws are closed to hold the biological tissue including the treatment target and its surrounding tissue.

3. The surgical device according to claim 1, wherein
each of the first holding sections has a first holding surface which is configured to hold the biological tissue as the treatment target,
each of the second holding sections has a second holding surface which is configured to hold the surrounding tissue of the biological tissue as the treatment target, and
the second holding surface is inclined with respect to the first holding surface.

4. The surgical device according to claim 1, wherein a second virtual plane defined midway between the second holding sections is present at a position shifted from a first virtual plane defined midway between the first holding sections.

5. The surgical device according to claim 1, wherein
the second holding sections have a first outer edge portion provided to the first jaw and a second outer edge portion provided to the second jaw, and
the first outer edge portion is arranged on at least one of the inner side and the outer side of the second outer edge portion, when the first and second jaws are closed to hold the biological tissue including the treatment target and its surrounding tissue.

6. The surgical device according to claim 1, wherein at least one of the second holding sections has a barrier function of preventing a fluid from flowing to the outside of the second holding sections, the fluid being produced from the biological tissue as the treatment target, when the energy is discharged from the energy discharge section to the biological tissue as the treatment target.

7. The surgical device according to claim 1, further comprising a groove which is provided between the first holding section and the second holding section of at least one of the first and second jaws, and which is configured to receive a fluid generated from the biological tissue as the treatment target, when the energy is discharged from the energy discharge section to the biological tissue as the treatment.

8. The surgical device according to claim 1, comprising a cooling section which is provided in the second holding section of at least one jaw provided with the energy discharge section of the first and second jaws, and configured to cool a fluid generated when the energy is applied to the biological tissue as the treatment target from the energy discharge section.
